(19) Europäisches Patentamt
European Patent Office
Office européen des brevets

(11) Veröffentlichungsnummer: **0 077 024**
**B1**

(12) # EUROPÄISCHE PATENTSCHRIFT

(45) Veröffentlichungstag der Patentschrift: **12.08.87**

(21) Anmeldenummer: **82109252.5**

(22) Anmeldetag: **07.10.82**

(51) Int. Cl.[4]: **C 07 D 403/04,** C 07 D 487/04, C 07 D 233/64, C 07 D 417/04, C 07 D 487/14, A 61 K 31/40, A 61 K 31/415, A 61 K 31/425, A 61 K 31/495, A 61 K 31/55 // (C07D487/04, 209:00, 209:00), (C07D487/14, 241:00, 235:00, 209:00),(C07D487/14, 243:00, 235:00, 209:00),(C07D487/14, 245:00, 235:00, 209:00)

(54) **Neue Imidazol-Derivate, Verfahren zu ihrer Herstellung und diese enthaltende pharmazeutische Präparate.**

(30) Priorität: **13.10.81 DE 3141063**

(43) Veröffentlichungstag der Anmeldung: **20.04.83 Patentblatt 83/16**

(45) Bekanntmachung des Hinweises auf die Patenterteilung: **12.08.87 Patentblatt 87/33**

(84) Benannte Vertragsstaaten: **AT BE CH DE FR GB IT LI LU NL SE**

(56) Entgegenhaltungen:
**EP-A-0 005 219**
**EP-A-0 058 890**
**CH-A-554 867**
**DE-A-2 155 558**
**US-A-3 708 598**

**K. HOFMANN "Imidazole and its derivatives, part I, (Monographie der Reihe "The chemistry of heterocyclic compounds") 1953, INTERSCIENCE PUBLISHERS, New York, London Seiten 33-45**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Klose, Walter, Dr., Beatestrasse 9g, D-1000 Berlin 27 (DE)**
Erfinder: **Böttcher, Irmgard, Dr., Frobenstrasse 46, CH- 4000 Basel (CH)**

**EP 0 077 024 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft neue Imidazol-Derivate der allgemeinen Formel I

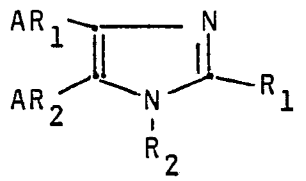

(I),

worin

$AR_1$ und $AR_2$ einen gegebenenfalls durch Halogenatome, Alkylreste mit 1 bis 4 Kohlenstoffatomen oder Alkoxyreste mit 1 bis 4 Kohlenstoffatomen substituierten Phenylrest bedeuten,

worin

$R_1$ einen gegebenenfalls durch Methylgruppen, Methoxycarbonylgruppen, Ethoxycarbonylgruppen, Ethoxycarbonylmethylgruppen, Ethoxycarbonylethylgruppen, Benzylgruppen oder Benzolsufonylgruppen substituierten Pyrrolylrest, Indolylrest, Imidazolylrest oder Thiazolylrest, oder einen 2,3-Dihydro-1H-pyrrolizinylrest darstellt und

$R_2$ ein Wasserstoffatom, eine gegebenenfalls durch ein Bromatom oder Jodatom substituierte Alkylgruppe mit 1 bis 6 Kohlenstoffatomen oder eine an das Stickstoffatom von $R_1$ gebundene Dimethylengruppe, Trimethylengruppe oder Tetramethylengruppe bedeutet.

Eine bevorzugte Gruppe von Imidazol-Derivaten der allgemeinen Formel I ist dadurch gekennzeichnet, daß die Substituenten $AR_1$ und $AR_2$ einen, gegebenenfalls in para-Stellung durch ein Fluoratom, ein Chloratom, eine 1 bis 4 Kohlenstoffatome enthaltende Alkoxygruppe substituierten Phenylrest darstellen. Derartige Imidazol-Derivate sind insbesondere solche, die dadurch gekennzeichnet sind, daß die Substituenten $AR_1$ und $AR_2$ einen Phenylrest, einen 4-Fluorphenylrest, einen 4-Chlorphenylrest, oder einen 4-Methoxyphenylrest darstellen.

Bevorzugt sind auch solche Imidazol-Derivate der allgemeinen Formel I, die dadurch gekennzeichnet sind, daß der Substituent $R_1$ ein gegebenenfalls durch Methylgruppen oder Methoxy- oder Ethoxycarbonylgruppen substituierter 2-Pyrrolylrest, 3-Pyrrolylrest, ein 7-[2,3-Dihydro-1H-pyrrolizidyl]-rest ein 2-Indolylrest, ein 2-Imidazolylrest oder ein 2-Thiazolylrest darstellt.

Die Erfindung betrifft ferner pharmazeutische Präparate, die ein oder zwei Imidazol-Derivate der allgemeinen Formel I enthalten.

Letztlich betrifft die Erfindung ein Verfahren zur Herstellung der Imidazol-Derivate der allgemeinen Formel I, das dadurch gekennzeichnet ist, daß man ein Diketon der allgemeinen Formel II

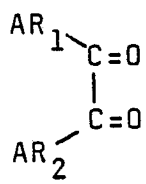

(II),

worin $AR_1$ und $AR_2$ die obengenannte Bedeutung besitzen in Gegenwart von Ammoniumionen mit einem Aldehyd der allgemeinen Formel III

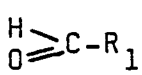

(III),

worin $R_1$ die obengenannte Bedeutung besitzt kondensiert und die erhaltenen Verbindungen gegebenenfalls N-alkyliert.

Erfindungsgemäß bedeuten die Substituenten $AR_1$ und $AR_2$ der Imidazol-Derivate jeweils einen gegebenenfalls durch Halogenatome, Alkylreste mit 1 bis 4 Kohlenstoffatomen oder Alkoxyreste mit 1 bis 4 Kohlenstoffatomen substituierten Phenylrest. Durch Halogenatome substituierte Phenylreste $AR_1$ und $AR_2$ sind beispielsweise Mono- oder Difluorphenylreste oder Mono- oder Dichlorphenylreste und insbesondere p-Fluorphenylreste oder p-Chlorphenylreste. Alkylsubstituierte Phenylreste sind beispielsweise solche, die Methyl-, Äthyl-, Propyl- oder Isopropylgruppen enthalten. Durch Alkoxygruppen substituierte Phenylreste sind beispielsweise solche, die Methoxy-, Äthoxy-, Propyloxy- oder Isopropyloxygruppen enthalten.

Die Phenylreste $AR_1$ und $AR_2$ können einfach oder mehrfach substituiert sein mit gleichen oder verschiedenen Substituenten. Vorzugsweise sind sie einfach substituiert. Bevorzugt ist insbesondere der p-Methoxyphenylrest.

Der Substituent $R_2$ der Imidazol-Derivate der allgemeinen Formel I ist erfindungsgemäß ein Wasserstoffatom oder ein gegebenenfalls durch ein Halogenatom (vorzugsweise Bromatom oder Jodatom) substituierter Alkylrest mit 1 bis 6 Kohlenstoffatomen oder eine an das Stickstoffatom von $R_1$ gebundene Dimethylengruppe, Trimethylengruppe oder Tetramethylengruppe. Bevorzugte halogensubstituierte Alkylreste sind beispielsweise die 2-Halogenäthylgruppe, die 3-Halogenpropylgruppe, und die 3-Halogenbutylgruppe. Diese Verbindungen sind vorzugsweise Zwischenprodukte.

Die Imidazol-Derivate der allgemeinen Formel I zeichnen sich durch eine ausgeprägte antiinflammatorische und antiallergische Wirksamkeit aus.

Darüberhinaus zeichnen sich die Imidazol-Derivate der allgemeinen Formel I dadurch aus, daß sie eine sehr günstige Dissoziation zwischen erwünschter pharmakologischer Wirksamkeit und unerwünschter - insbesondere ulcerogenen - Nebenwirkungen besitzen.

Die antiinflammatorische Wirksamkeit der erfindungsgemäßen Substanzen kann mit Hilfe des bekannten Adjuvans-Arthritis-Testes ermittelt werden, der wie folgt durchgeführt wird:

Es werden weibliche und männliche Ratten des Stammes Lewis (LEW) in der Gewichtsspanne zwischen 110-190 g verwendet. Die Tiere erhalten Trinkwasser und Altromin- Preßfutter ad libitum.

Für jede Dosisgruppe werden 10 Ratten eingesetzt.

Mycobacterium butyricum der Firma Difko, Detroit wird als Reizmittel verwandt. Eine Suspension von 0,5 mg Mycobacterium butyricum in 0,1 ml dünnflüssigem Paraffin (DAB 7) wird in die rechte Hinterpfote subplantar injiziert.

Die Testsubstanzen werden vom 11. Versuchstag an täglich über 4 Tage oral gegeben. Die Substanzen werden als klare wässrige Lösung oder als Kristallsuspension unter Zusatz von Myrj 53 (85 mg %) in isotonischer Natriumchlorid-Lösung verabreicht.

Versuchsansatz:

Die Ratten werden in bezug auf ihr Körpergewicht möglichst gleichmäßig in verschiedene Gruppen eingeteilt. Nach plethysmographischer Volumenmessung der rechten Hinterpfote wird in diese subplantar 0,1 ml Adjuvans injiziert.

Die rechten Hinterpfoten werden vom 14. Versuchstag bis zu Versuchsende gemessen. Die Versuchsdauer beträgt drei Wochen.

Bestimmt wird die Dosis bei der eine 40 %ige Abnahme des Pfotenvolumens im Vergleich zum unbehandleten Tier erzielt wird ($ED_{40}$ in mg/kg Körpergewicht).

Die nachfolgende Tabelle zeigt die in diesem Test erhaltenen Ergebnisse der erfindungsgemäßen Verbindungen im Vergleich zu der aus der DE-A-21 55 558 vorbekannten Verbindung 1. Diese Substanz ist unter den vorbekannten Verbindungen jene, die denjenigen der Erfindung am strukturanalogsten ist. Die Tabelle zeigt, daß die erfindungsgemäßen Verbindungen signifikant stärker wirksam sind als dieses vorbekannte 4,5-Bis-(4-methoxyphenyl)-2-(2-thienyl)-imidazol.

| Nr. | Substanz | Adjuvans-Arthritis- Test Dosis in mg/kg Tier | % Hemmung |
|---|---|---|---|
| 1 | 4,5-Bis-(4-methoxyphenyl)-2-(2-thienyl)-imidazol | 4 x 10 | 12 |
| 2 | 4,5-Bis-(4-methoxyphenyl)-2-(2-pyrrolyl)-imidazol | 4 x 10 | 39 |
| 3 | 4,5-Bis-(4-methoxyphenyl)-2-(4-methoxycarbonyl-2--pyrrolyl)-imidazol | 4 x 10 | 30 |
| 4 | 4,5-Bis-(4-fluorphenyl)-2-(2-pyrrolyl)-1-(3--brompropyl)-imidazol | 4 x 10 | 21 |
| 5 | 7-[4,5-Bis-(4-methoxyphenyl)-2-imidazolyl]-2,3--dihydro-1H-pyrrolizin | 4 x 10 | 31 |
| 6 | 2,3-Bis-(4-methoxyphenyl)-5,6-dihydroimidazo--[1,2-a]-pyrrolo-[2,1-c]-pyrazin | 4 x 10 | 25 |
| 7 | 2,3-Bis-(4-methoxyphenyl)-6,7-dihydro-5H-imidazo--[1,2-a]-pyrrolo-1,4-diazepin | 4 x 10 | 34 |
| 8 | 2,3-Bis-(4-methoxyphenyl)-5,6,7,8-tetrahydroimidazo-[1,2-a]-pyrrolo-[2,1-c]-1,4-diazocin | 4 x 10 | 20 |

Somit eignen sich die neuen Verbindungen in Kombination mit den in der galenischen Pharmazie üblichen Trägermitteln zur Behandlung zum Beispiel von akuter chronischer Polyarthritis, Neurodermitis, Asthma bronchiale, Heufieber u.a..

Bemerkenswert ist ferner, daß sich die Imidazol-Derivate der allgemeinen Formel I gemäß Anspruch 1 darüberhinaus auch zur Behandlung von Migräne und von Dysmenorhoe eignen.

Die Herstellung der Arzneimittelspezialitäten erfolgt in üblicher Weise, indem man die Wirkstoffe mit geeigneten Zusätzen, Trägersubstanzen und Geschmackskorrigentien in die gewünschten Applikationsformen wie Tabletten, Dragees, Kapseln, Lösungen, Inhalationsmitteln usw. überführt.

Für die orale Anwendung eignen sich insbesondere Tabletten, Dragees und Kapseln, welche beispielsweise 1 bis 250 mg Wirkstoff und 50 mg bis 2 g pharmakologisch unwirksamen Trägers, wie zum Beispiel Laktose, Amylose, Talkum, Gelatine, Magnesiumstearat und ähnliches, sowie die üblichen Zusätze enthalten.

Die neuen Imidazol-Derivate der allgemeinen Formel I gemäß Anspruch 1 können nach an sich bekannten Verfahren hergestellt werden. Ein geeignetes Herstellungsverfahren ist beispielsweise die im Anspruch 39 beschriebene Synthese.

Diese Synthese kann unter an sich bekannten Bedingungen durchgeführt werden. (Arnold Weissberger: The Chemistry of Heterocyclic Compounds, Vol 6: Klaus Hoffmann: Imidazole and its Derivatives Pakt I - Interscience Publishers Inc. New York, 1953, Seite 34 ff.)

Die Ausgangsverbindungen für das erfindungsgemäße Verfahren gemäß Anspruch 39 sind bekannt oder können in an sich bekannter Weise hergestellt werden. (Chem. Ber. 113, 1980, 2694; Canad. J. Chem. 56, 1978, 654 oder J. Chem. Soc. 84, 1962, 635).

Die nachfolgenden Ausführungsbeispiele dienen zur Erläuterung des erfindungsgemäßen Verfahrens.

**Beispiel 1**

Eine Mischung aus 18,7 g 4,4'-Dimethoxy-benzil, 10,0 g 2-Formylpyrrol 50,0 g Ammoniumacetat und 200 ml Essigsäure werden im Kolben in ein auf 170°C vorgeheiztes Ölbad gestellt und 15 Minuten gerührt. Dann wird in der Wärme soviel Wasser zugesetzt, daß ein bleibender Niederschlag bestehen bleibt und dieses Gemisch wird dann über Nacht stehengelassen. Der ausgefallene Niederschlag wird abfiltriert und mittels Chromatographie an Kieselgel, Laufmittel Hexan/Essigsäureethylester (1:1), getrennt.

Man erhält 11,2 g 4,5-Bis-(4-methoxyphenyl)-2-(2-pyrrolyl)-imidazol vom Schmelzpunkt 237°C.

$C_{21}H_{19}N_3O_2$ (345,407)
Ber.: C 73,02 H 5,54 N 12,17
Gef.: C 72,90 H 5,50 N 11,87

**Beispiel 2**

Die Darstellung von 4,5-Bis-(4-Chlorphenyl)-2-(2-pyrrolyl)-imidazol erfolgt analog Beispiel 1 durch Umsetzung von 4,4'-Dichlorbenzil mit 2-Formylpyrrol. Schmelzpunkt 315°C.

$C_{19}H_{13}Cl_2N_3$ (354,251)
Ber.: C 64,42 H 3,70 N 11,86 Cl 20,02
Gef.: C 64,58 H 3,71 N 11,42 Cl 20,31

**Beispiel 3**

Die Darstellung von 4,5-Bis-(4-fluorphenyl)-2-(2-pyrrolyl)-imidazol erfolgt analog Beispiel 1 durch Umsetzung von 4,4'-Difluorbenzil mit 2-Formylpyrrol. Schmelzpunkt 276° C.

$C_{19}H_{13}F_2N_3$ (321,33)
Ber.: C 71,02 H 4,08 N 13,08 F 11,82
Gef.: C 70,79 H 4,12 N 12,93 F 11,45

**Beispiel 4**

Die Darstellung von 4,5-Bis-(4-methoxyphenyl)-2-(1-methyl-2-pyrrolyl)-imidazol erfolgt nach Beispiel 1 durch Umsetzung von 4,4'-Dimethoxybenzil mit 1-Methyl-2-formylpyrrol. Schmelzpunkt 110° C.

$C_{22}H_{21}N_3O_2$ (359,4)
Ber.: C 73,52 H 5,89 N 11,69
Gef.: C 73,24 H 6,05 N 11,36

**Beispiel 5**

Die Darstellung von 4,5-Bis-(4-methoxyphenyl)-2-(3-ethoxy-carbonyl-2-pyrrolyl)-imidazol erfolgt analog Beispiel 1 durch Umsetzung von 4,4'-Dimethoxybenzil mit 2-Formyl-3-ethoxycarbonylpyrrol. Schmelzpunkt 193° C.

$C_{24}H_{23}N_3O_4$ (417,5)
Ber.: C 69,03 H 5,55 N 10,05
Gef.: C 68,86 H 5,98 N 10,16

**Beispiel 6**

Die Darstellung von 4,5-Bis-(4-methoxyphenyl)-2-(4-methoxy-carbonyl-2-pyrrolyl)-imidazol erfolgt analog Beispiel 1 durch Umsetzung von 4,4'-Dimethoxybenzil mit 2-Formyl-4-methoxycarbonylpyrrol. Schmelzpunkt 236° C.

$C_{23}H_{21}N_3O_4$ (403,4)
Ber.: C 68,47 H 5,25 N 10,42
Gef.: C 68,51 H 5,18 N 10,12

Herstellung des Ausgangsmaterials:

3,6 g (27 m mol)-2-Cyanopyrrol-4-carbonsäuremethylester, 17 g Raney-Nickel und 450 ml 75 %ige Ameisensäure werden im Kolben in ein auf 120°C vorgeheiztes Ölbad gebracht und 1 Stunde zur Reaktion gebracht. Anschließend wird in 1 l Eiswasser gegeben und mehrmals mit Ether extrahiert. Nach dem Trocknen und Einengen der Etherphase erhält man 1,4 g (36 % der Theorie) 2-Formylpyrrol-4-carbonsäuremethylester mit dem Schmelzpunkt 126° C.

**Beispiel 7**

Die Darstellung von 4,5-Bis-(4-methoxyphenyl)-2-(1-benzyl-2-pyrrolyl)-imidazol erfolgt analog Beispiel 1 durch Umsetzung von 4,4'-Dimethoxybenzil mit 1-Benzyl-2-formylpyrrol. Schmelzpunkt 183° C.

$C_{28}H_{25}N_3O_2$ (435,5)
Ber.: C 77,21 H 5,78 N 9,64
Gef.: C 77,03 H 5,93 N 9,01

**Beispiel 8**

Die Darstellung von 4,5-Bis-(4-methoxyphenyl)-2-(1-phenyl-sulfonyl-2-pyrrolyl)-imidazol erfolgt analog Beispiel 1 durch Umsetzung von 4,4'-Dimethoxybenzil mit 1-phenylsulfonyl-2-formylpyrrol. Schmelzpunkt 135° C.

$C_{27}H_{23}N_3O_4S$ (485,6)
Ber.: C 66,79 H 4,77 N 8,65 S 6,60

Gef.: C 66,81 H 4,45 N 8,52 S 6,43

**Beispiel 9**

Die Darstellung von 4,5-Bis-(4-methoxyphenyl)-2-(3-pyrrolyl)-imidazol erfolgt analog Beispiel 1 durch Umsetzung von 4,4'-Dimethoxybenzil mit 3-Formylpyrrol. Schmelzpunkt 232° C.
$C_{21}H_{19}N_3O_2$ (345,4)
Ber.: C 73,02 H 5,54 N 12,17
Gef.: C 73,44 H 5,56 N 11,84

**Beispiel 10**

Die Darstellung von 4,5-Bis-(4-methoxyphenyl)-2-(2-ethoxycarbonyl-3-pyrrolyl)-imidazol erfolgt analog Beispiel 1 durch Umsetzung von 4,4'-Dimethoxybenzil mit 2-Ethoxycarbonyl-3-formylpyrrol. Schmelzpunkt 176° C.
$C_{24}H_{23}N_3O_4$ (417,4)
Ber.: C 69,05 H 5,55 N 10,07
Gef.: C 69,26 H 5,45 N 9,85

**Beispiel 11**

Die Darstellung von 2-Ethoxycarbonyl-4-[4,5-bis-(4-methoxyphenyl)-2-imidazolyl]-5-methylpyrrol-3-essigsäureethylester erfolgt analog Beispiel 1 durch Umsetzung von 4,4'-Dimethoxy-benzil mit 2-Ethoxycarbonyl-4-formyl-5-methyl-3-pyrrolessigsäureethylester. Schmelzpunkt 186° C.
$C_{29}H_{31}N_3O_6$ (517,6)
Ber.: C 67,30 H 6,04 N 8,12
Gef.: C 67,06 H 6,22 N 7,96

**Beispiel 12**

Die Darstellung von 4,5-Bis-(4-methoxyphenyl)-2-(3,4,5-trimethyl-2-pyrrolyl)-imidazol erfolgt analog Beispiel 1 durch Umsetzung von 4,4'-Dimethoxybenzil mit 3,4,5-Trimethyl-2-formylpyrrol. Schmelzpunkt 115° C.
$C_{24}H_{25}N_3O_2$ (387,5)
Ber.: C 74,39 H 6,50 N 10,85
Gef.: C 74,10 H 6,34 N 10,93

**Beispiel 13**

Die Darstellung von 4,5-Bis-(4-methoxyphenyl)-2-(3,4-dimethyl-2-pyrrolyl)-imidazol erfolgt analog Beispiel 1 durch Umsetzung von 4,4'-Dimethoxybenzil mit 3,4-Dimethyl-2-formylpyrrol. Schmelzpunkt 134° C.
$C_{23}H_{23}N_3O_2$ (373,5)
Ber.: C 73,97 H 6,21 N 11,25
Gef.: C 73,78 H 6,30 N 11,02

**Beispiel 14**

Die Darstellung von 2-Ethoxycarbonyl-4-[4,5-bis-(4-methoxyphenyl)-2-imidazolyl]-5-methylpyrrol-3-propionsäureethylester erfolgt analog Beispiel 1 durch Umsetzung von 4,4'-Dimethoxybenzyl mit 2-Ethoxycarbonyl-4-formyl-5-methylpyrrol-3-propionsäureethylester. Schmelzpunkt 90° C.
$C_{30}H_{33}N_3O_6$ (531,6)
Ber.: C 67,78 H 6,26 N 7,90
Gef.: C 67,45 H 6,46 N 7,83

**Beispiel 15**

Die Darstellung von 7-[4,5-Bis-(4-methoxyphenyl)-2-imidazolyl]-2,3-dihydro-1H-pyrrolizin erfolgt analog Beispiel 1 durch Umsetzung von 4,4'-Dimethoxybenzil mit 7-Formyl-2,3-dihydro-1H-pyrrolizin. Schmelzpunkt 238° C.

$C_{24}H_{23}N_3O_2$ (385,4)
Ber.: C 74,78 H 6,01 N 10,90
Gef.: C 74,60 H 6,11 N 10,64

Herstellung des Ausgangsmaterials:

13,21 g (0,1 mol) 7-Cyano-2,3-dihydro-1H-pyrrolizin werden in 150 ml absolutem Toluol gelöst und auf -20° C abgekühlt. Zu dieser Lösung werden 108 ml (0,13 mol) einer 1,2 molaren Diisobutylaluminiumhydridlösung in Toluol getropft, dann wird auf Raumtemperatur erwärmt, 1 Stunde nachgerührt und mit 300 ml 10 %iger wässriger Citronensäurelösung zersetzt. Es wird mit Methylenchlorid extrahiert, die organische Phase getrocknet, eingeengt und der Rückstand aus Ether umkristallisiert. Man erhält 8,0 g (59 % der Theorie) 7-Formyl-2,3-dihydro-1H-pyrrolizin mit Schmelzpunkt 58° C.

**Beispiel 16**

Die Darstellung von 4,5-Bis-(4-methoxyphenyl)-2-(2-indolyl)-imidazol erfolgt analog Beispiel 1 durch Umsetzung von 4,4'-Dimethoxybenzil mit 2-Formylindol. Schmelzpunkt 130° C.

$C_{25}H_{21}N_3O_2$ (395,5)
Ber.: C 75,93 H 5,35 N 10,63
Gef.: C 75,61 H 5,50 N 10,38

**Beispiel 17**

Die Darstellung von 4,5-Bis-(4-methoxyphenyl)-2-(3-indolyl)-imidazol erfolgt analog Beispiel 1 durch Umsetzung von 4,4'-Dimethoxybenzil mit 3-Formylindol. Schmelzpunkt 246° C.

$C_{25}H_{21}N_3O_2$ (395,5)
Ber.: C 75,93 H 5,35 N 10,63
Gef.: C 75,81 H 5,70 N 10,49

**Beispiel 18**

Die Darstellung von 4,5-Bis-(4-methoxyphenyl)-2-(2-imidazolyl)-imidazol erfolgt analog Beispiel 1 durch Umsetzung von 4,4'-Dimethoxybenzil mit 2-Formylimidazol. Schmelzpunkt 178° C.

$C_{20}H_{18}N_4O_2$ (346,42)
Ber.: C 69,35 H 5,24 N 16,18
Gef.: C 69,51 H 4,99 N 16,30

**Beispiel 19**

Die Darstellung von 4,5-Bis-(4-methoxyphenyl)-2-(1-methyl-2-imidazolyl)-imidazol erfolgt analog Beispiel 1 durch Umsetzung von 4,4'-Dimethoxybenzil mit 1-Methyl-2-formylimidazol. Schmelzpunkt 196° C.

$C_{21}H_{20}N_4O_2$ (360,4)
Ber.: C 69,98 H 5,59 N 15,55
Gef.: C 69,78 H 5,58 N 15,43

**Beispiel 20**

Die Darstellung von 4,5-Bis-(4-methoxyphenyl)-2-(1-benzyl-2-imidazolyl)-imidazol erfolgt analog Beispiel 1 durch Umsetzung von 4,4'-Dimethoxybenzil mit 1-Benzyl-2-formylimidazol. Schmelzpunkt 180° C.

$C_{27}H_{24}N_4O_2$ (436,4)
Ber.: C 74,29 H 5,54 N 12,82
Gef.: C 73,92 H 5,71 N 12,63

**Beispiel 21**

Die Darstellung von 4,5-Bis-(4-methoxyphenyl)-2-(2-thiazolyl)-imidazol erfolgt analog Beispiel 1 durch Umsetzung von 4,4'-Dimethoxybenzil mit 2-Formylthiazol. Schmelzpunkt 199° C.

$C_{20}H_{17}N_3O_2S$ (363,2)
Ber.: C 66,08 H 4,72 N 11,57 S 8,83
Gef.: C 66,04 H 5,01 N 11,39 S 8,59

**Beispiel 22**

1,61 g 4,5-Bis-(4-methoxyphenyl)-2-(2-pyrrolyl)-imidazol, 6,29 g Dibromethan und 1,5 g Ethyl-diisopropylamin werden in 120 ml Acetonitril gelöst und 48 Stunden zum Rückfluß erhitzt. Dann wird die Reaktionslösung im Vakuum zur Trockne eingeengt und das 4,5-Bis-(4-methoxyphenyl)-2-(2-pyrrolyl)-1-(2-bromethyl)-imidazol durch Chromatographie an Kieselgel mit Hexan/Essigester (1:1) getrennt, erhalten werden 0,5 g. Schmelzpunkt 135° C.

$C_{23}H_{22}N_3O_2Br$ (452,349)
Ber.: C 61,06 H 4,90 N 9,29 Br 17,68
Gef.: C 60,95 H 4,85 N 9,32 Br 17,40

**Beispiel 23**

Die Darstellung von 4,5-Bis-(4-Fluorphenyl)-2-(2-pyrrolyl)-1-(3-brompropyl)-imidazol erfolgt analog Beispiel 22 durch Umsetzung von 4,5-Bis-(4-fluorphenyl)-2-(2-pyrrolyl)-imidazol mit 1,3-Dibrompropan. Schmelzpunkt 157° C.

$C_{22}H_{20}F_2N_3Br$ (442,301)
Ber.: C 59,74 H 4,10 N 9,50 F 8,59 Br 18,07
Gef.: C 59,60 H 4,22 N 9,39 F 8,44 Br 18,01

**Beispiel 24**

Die Darstellung von 4,5-Bis-(4-methoxyphenyl)-2-(2-pyrrolyl)-1-(4-jodbutyl)-imidazol erfolgt analog Beispiel 22 durch Umsetzung von 4,5-Bis-(4-methoxyphenyl)-2-(2-pyrrolyl)-imidazol mit 1,4-Dijodbutan. Schmelzpunkt 85° C.

$C_{25}H_{26}N_3O_2J$ (527,407)
Ber.: C 56,94 H 4,97 N 7,97 J 24,06
Gef.: C 57,20 H 5,03 N 7,48 J 23,74

**Beispiel 25**

Die Darstellung von 4,5-Bis-(4-methoxyphenyl)-2-(2-pyrrolyl)-1-(3-brompropyl)-imidazol erfolgt analog Beispiel 22 durch Umsetzung von 4,5-Bis-(4-methoxyphenyl)-2-(2-pyrrolyl)-imidazol mit 1,3-Dibrompropan. Schmelzpunkt 97° C.

$C_{24}H_{24}N_3O_2Br$ (466,4)
Ber.: C 61,18 H 5,07 N 8,26 Br 15,42
Gef.: C 61,31 H 5,19 N 8,43 Br 15,70

**Beispiel 26**

Die Darstellung von 4,5-Bis-(4-methoxyphenyl)-2-(2-pyrrolyl)-1-butylimidazol erfolgt analog Beispiel 22 durch Umsetzung von 4,5-Bis-(4-methoxyphenyl)-2-(2-pyrrolyl)-imidazol mit Brombutan. Schmelzpunkt 76° C.

$C_{25}H_{27}N_3O_2$ (401,5)
Ber.: C 74,79 H 6,78 N 10,47
Gef C 74,51 H 7,00 N 10,28

**Beispiel 27**

Die Darstellung von 4,5-Bis-(4-methoxyphenyl)-2-(2-pyrrolyl)-1-methylimidazol erfolgt analog Beispiel 22 durch Umsetzung von 4,5-Bis-(4-methoxyphenyl)-2-(2-pyrrolyl)-imidazol mit Jodmethan. Schmelzpunkt 134° C.
$C_{22}H_{21}N_3O_2$ (359,4)
Ber.: C 73,52 H 5,89 N 11,69
Gef.: C 73,61 H 5,80 N 11,49

**Beispiel 28**

0,760 g 4,5-Bis-(4-methoxyphenyl)-2-(2-pyrrolyl)-1-(2-bromethyl)-imidazol werden in 20 ml Dimethylformamid gelöst, mit 0,15 g Natriumhydrid (55 %ig in Weißöl) versetzt und 1 Stunde bei 60°C gerührt. Die Reaktionsmischung wird in Eiswasser gegossen, mit Essigsäureethylester extrahiert, und durch Chromatographie an Kieselgel, Laufmittel Essigsäureethylester/Hexan (2:1) getrennt. Erhalten werden 0,400 g 2,3-Bis-(4-methoxyphenyl)-5,6-dihydroimidazo-[1,2-a]-pyrrolo-[2,1-c]-pyrazin vom Schmelzpunkt 172° C.
$C_{23}H_{21}N_3O_2$ (371,437)
Ber.: C 74,37 H 5,71 N 11,31
Gef.: C 74,07 H 5,90 N 10,85

**Beispiel 29**

1,4 g 4,5-Bis-(4-methoxyphenyl)-2-(2-pyrrolyl)-1-(3-brom-propyl)-imidazol werden analog Beispiel 28 zu 0,6 g 2,3-Bis-(4-methoxyphenyl)-6,7-dihydro-5H-imidazo[1,2-a]-pyrrolo-[2,1-c]-[1,4]-diazepin cyclisiert. Schmelzpunkt 140° C.

**Beispiel 30**

1,53 g 4,5-Bis-(4-methoxyphenyl)-2-(2-pyrrolyl)-1-(4-jodbutyl)-imidazol werden analog Beispiel 28 zu 0,85 g 2,3-Bis-(4-methoxyphenyl)-5,6,7,8-tetrahydroimidazo-[1,2-a]-pyrrolo-[2,1-c]-[1,4]-diazocin vom Schmelzpunkt 181° C cyclisiert.
$C_{25}H_{25}N_3O_2$ (399,5)
Ber.: C 75,16 H 6,31 N 10,52
Gef.: C 75,41 H 6,48 N 10,34

**Patentansprüche**

1. Imidazol-Derivate der allgemeinen Formel I

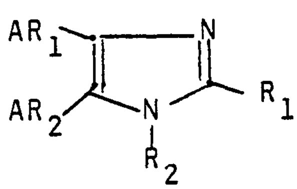

(I),

worin
$AR_1$ und $AR_2$ einen gegebenenfalls durch Halogenatome, Alkylreste mit 1 bis 4 Kohlenstoffatomen oder Alkoxyreste mit 1 bis 4 Kohlenstoffatomen substituierten Phenylrest bedeuten,
worin
$R_1$ einen gegebenenfalls durch Methylgruppen, Methoxycarbonylgruppen, Ethoxycarbonylgruppen, Ethoxycarbonylmethylgruppen, Ethoxycarbonylethylgruppen, Benzylgruppen oder Benzolsufonylgruppen substituierten Pyrrolylrest, Indolylrest, Imidazolylrest oder Thiazolylrest, oder einen 2,3-Dihydro-1H-pyrrolizinylrest darstellt und
$R_2$ ein Wasserstoffatom, eine gegebenenfalls durch ein Halogenatom substituierte Alkylgruppe mit 1 bis 6 Kohlenstoffatomen oder eine an das Stickstoffatom von $R_1$ gebundene Dimethylengruppe, Trimethylengruppe

oder Tetramethylengruppe bedeutet.

2. Imidazol-Derivate der allgemeinen Formel I gemäß Anspruch 1, dadurch gekennzeichnet, daß die Substituenten $AR_1$ und $AR_2$ einen, gegebenenfalls in para-Stellung durch ein Fluoratom, ein Chloratom, eine 1 bis 4 Kohlenstoffatome enthaltende Alkoxygruppe substituierten Phenylrest darstellen.

3. Imidazol-Derivate der allgemeinen Formel I gemäß Anspruch 2, dadurch gekennzeichnet, daß die Substituenten $AR_1$ und $AR_2$ einen Phenylrest, einen 4-Fluorphenylrest, einen 4-Chlorphenylrest, oder einen 4-Methoxyphenylrest darstellen.

4. Imidazol-Derivate der allgemeinen Formel I gemäß Anspruch 1 bis 3, dadurch gekennzeichnet, daß der Substituent $R_1$ ein gegebenenfalls durch Methylgruppen oder Methoxy- oder Ethoxycarbonylgruppen substituierter 2-Pyrrolylrest, 3-Pyrrolylrest, ein 7-[2,3-Dihydro-1H-pyrrolizidyl]-rest ein 2-Indolylrest, ein 2-Imidazolylrest oder ein 2-Thiazolylrest darstellt.

5. 4,5-Bis-(4-methoxyphenyl)-2-(2-pyrrolyl)-imidazol.

6. 4,5-Bis-(4-chlorphenyl)-2-(2-pyrrolyl)-imidazol.

7. 4,5-Bis-(4-fluorphenyl)-2-(2-pyrrolyl)-imidazol.

8. 4,5-Bis-(4-methoxyphenyl)-2-(1-methyl-2-pyrrolyl)-imidazol.

9. 4,5-Bis-(4-methoxyphenyl)-2-(3-ethoxycarbonyl-2-pyrrolyl)-imidazol.

10. 4,5-Bis-(4-methoxyphenyl)-2-(4-methoxycarbonyl-2-pyrrolyl)-imidazol.

11. 4,5-Bis-(4-methoxyphenyl)-2-(1-benzyl-2-pyrrolyl)-imidazol.

12. 4,5-Bis-(4-methoxyphenyl)-2-(1-phenylsulfonyl-2-pyrrolyl)-imidazol.

13. 4,5-Bis-(4-methoxyphenyl)-2-(3-pyrrolyl)-imidazol.

14. 4,5-Bis-(4-methoxyphenyl)-2-(2-ethoxycarbonyl-3-pyrrolyl)-imidazol.

15. 2-Ethoxycarbonyl-4-[4,5-bis-(4-methoxyphenyl)-2-imidazolyl]-5-methyl-pyrrol-3-essigsäure-ethylester.

16. 4,5-Bis-(4-methoxyphenyl)-2-(3,4,5-trimethyl-2-pyrrolyl)-imidazol.

17. 4,5-Bis-(4-methoxyphenyl)-2-(3,4-dimethyl-2-pyrrolyl)-imidazol.

18. 2-Ethoxycarbonyl-4-[4,5-bis-(4-methoxyphenyl)-2-imidazolyl]-5-methyl-pyrrol-3-propionsäure-ethylester.

19. 7-[4,5-Bis-(4-methoxyphenyl)-2-imidazolyl]-2,3-dihydro-1H-pyrrolizin.

20. 4,5-Bis-(4-methoxyphenyl)-2-(2-indolyl)-imidazol.

21. 4,5-Bis-(4-methoxyphenyl)-2-(3-indolyl)-imidazol.

22. 4,5-Bis-(4-methoxyphenyl)-2-(2-imidazolyl)-imidazol.

23. 4,5-Bis-(4-methoxyphenyl)-2-(1-methyl-2-imidazolyl)-imidazol.

24. 4,5-Bis-(4-methoxyphenyl)-2-(1-benzyl-2-imidazolyl)-imidazol.

25. 4,5-Bis-(4-methoxyphenyl)-2-(2-thiazolyl)-imidazol.

26. 4,5-Bis-(4-methoxyphenyl)-2-(2-pyrrolyl)-1-(2-bromethyl)-imidazol.

27. 4,5-Bis-(4-fluorphenyl)-2-(2-pyrrolyl)-1-(3-brompropyl)-imidazol.

28. 4,5-Bis-(4-methoxyphenyl)-2-(2-pyrrolyl)-1-(4-jodbutyl)-imidazol.

29. 4,5-Bis-(4-methoxyphenyl)-2-(2-pyrrolyl)-1-(3-brompropyl)-imidazol.

30. 4,5-Bis-(4-methoxyphenyl)-2-(2-pyrrolyl)-1-butyl-imidazol.

31. 4,5-Bis-(4-methoxyphenyl)-2-(2-pyrrolyl)-1-methyl-imidazol.

32. 2,3-Bis-(4-methoxyphenyl)-5,6-dihydro-imidazo-[1,2-a]-pyrrolo-[2,1-c]-pyrazin.

33. 2,3-Bis-(4-methoxyphenyl)-6,7-dihydro-5H-imidazo-[1,2-a]-pyrrolo-[2,1-c]-1,4-diazepin.

34. 2,3-Bis-(4-methoxyphenyl)-5,6,7,8-tetrahydroimidazo-[1,2-a]-pyrrolo-[2,1-c]-1,4-diazocin.

35. Pharmazeutische Präparate, enthaltend ein oder zwei Imidazol-Derivate gemäß Anspruch 1 bis 34.

36. Pharmazeutische Präparate gemäß Anspruch 35 zur Behandlung entzündlicher oder allergischer Erkrankungen des Menschen.

37. Pharmazeutische Präparate gemäß Anspruch 35 zur Behandlung von Migräne.

38. Pharmazeutische Präparate gemäß Anspruch 35 zur Behandlung von Dysmenorhoe.

39. Verfahren zur Herstellung von Imidazol-Derivaten der allgemeinen Formel I

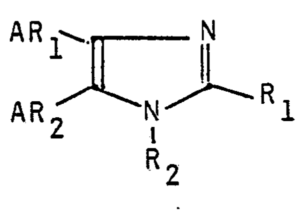

(I),

worin

$AR_1$, $AR_2$, $R_1$ und $R_2$ die im Patentanspruch 1 angegebene Bedeutung besitzen, dadurch gekennzeichnet, daß man ein Diketon der allgemeinen Formel II

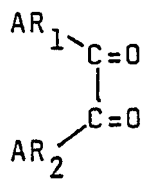

(II),

worin $AR_1$ und $AR_2$ die obengenannte Bedeutung besitzen in Gegenwart von Ammoniumionen mit einem Aldehyd der allgemeinen Formel

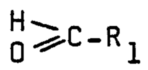

(III),

worin $R_1$ die obengenannte Bedeutung besitzt kondensiert und die erhaltenen Verbindungen gegebenenfalls N-alkyliert.

**Claims**

1. Imidazole derivatives of general formula I

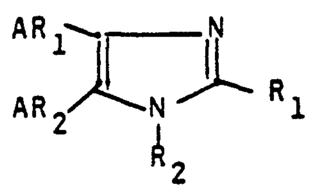

(I)

wherein

$AR_1$ and $AR_2$ are each a phenyl group, optionally substituted by halogen atoms, alkyl groups of 1 to 4 carbon atoms or alkoxy groups of 1 to 4 carbon atoms,

$R_1$ is a pyrrolyl, indolyl, imidazolyl or thiazolyl group, optionally substituted by methyl groups, methoxycarbonyl groups, ethoxycarbonylmethyl groups, ethoxycarbonylethyl groups, benzyl groups or benzenesulphonyl groups, or is a 2,3-dihydro-1H-pyrrolizinyl group, and

$R_2$ is a hydrogen atom, an alkyl group of 1 to 6 carbon atoms, optionally substituted by a halogen atom or, bound to the nitrogen atom from $H_1$, is a dimethylene, trimethylene or tetramethylene group.

2. Imidazole derivatives of general formula 1 according to claim 1, characterised in that the substituents $AR_1$ and $AR_2$ are each a phenyl group, optionally substituted in the para-position by a fluorine atom, a chlorine atom or an alkoxy group of 1 to 4 carbon atoms.

3. Imidazole derivatives of general formula 1 according to claim 2, characterised in that the substituents $AR_1$ and $AR_2$ are phenyl, 4-fluorophenyl, 4-chlorophenyl or 4-methoxyphenyl.

4. Imidazole derivatives of general formula 1 according to claims 1 to 3, characterised in that the substituent $R_1$ is a 2-pyrrolyl or 3-pyrrolyl group, optionally substituted by methyl groups or methoxy- or ethoxycarbonyl groups, a 7-(2,3-dihydro-1H-pyrrolizidyl), a 2-indolyl, a 2-imidazolyl or a 2-thiazolyl group.

5. 4,5-Bis-(4-methoxyphenyl)-2-(2-pyrrolyl)imidazole.

6. 4,5-Bis-(4-chlorophenyl)-2-(2-pyrrolyl)imidazole.

7. 4,5-Bis-(4-fluorophenyl)-2-(2-pyrrolyl)imidazole.

8. 4,5-Bis-(4-methoxyphenyl)-2-(1-methyl-2-pyrrolyl)imidazole.

9. 4,5-Bis-(4-methoxyphenyl)-2-(3-ethoxycarbonyl-2-pyrrolyl)imidazole.

10. 4,5-Bis-(4-methoxyphenyl)-2-(4-methoxycarbonyl-2-pyrrolyl)imidazole.

11. 4,5-Bis-(4-methoxyphenyl)-2-(1-benzyl-2-pyrrolyl)imidazole.

12. 4,5-Bis-(4-methoxyphenyl)-2-(1-phenylsulphonyl-2-pyrrolyl)imidazole.

13. 4,5-Bis-(4-methoxyphenyl)-2-(3-pyrrolyl)imidazole.

14. 4,5-Bis-(4-methoxyphenyl)-2-(2-ethoxycarbonyl-3-pyrrolyl)imidazole.
15. Ethyl 2-ethoxycarbonyl-4-[4,5-bis-(4-methoxyphenyl)-2-imidazolyl]-methylpyrrole-3-acetate.
16. 4,5-Bis-(4-methoxyphenyl)-2-(3,4,5-trimethyl-2-pyrrolyl)imidazole.
17. 4,5-Bis-(4-methoxyphenyl)-2-(3,4-dimethyl-2-pyrrolyl)imidazole.
18. Ethyl 2-ethoxycarbonyl-4-[4,5-bis-(4-methoxyphenyl)-2-imidazolyl]-5-methylpyrrole-3-propionate.
19. 7-[4,5-Bis-(4-methoxyphenyl)-2-imidazolyl]-2,3-dihydro-1H-pyrrolizine.
20. 4,5-Bis-(4-methoxyphenyl)-2-(2-indolyl)imidazole.
21. 4,5-Bis-(4-methoxyphenyl)-2-(3-indolyl)imidazole.
22. 4,5-Bis-(4-methoxyphenyl)-2-(2-imidazolyl)imidazole.
23. 4,5-Bis-(4-methoxyphenyl)-2-(1-methyl-2-imidazolyl)imidazole.
24. 4,5-Bis-(4-methoxyphenyl)-2-(1-benzyl-2-imidazolyl)imidazole.
25. 4,5-Bis-(4-methoxyphenyl)-2-(2-thiazolyl)imidazole.
26. 4.5-Bis-(4-methoxyphenyl)-2-(2-pyrrolyl)-1-(2-bromoethyl)imidazole.
27. 4,5-Bis-(4-fluorophenyl)-2-(2-pyrrolyl)-1-(3-bromopropyl)imidazole.
28. 4,5-Bis-(4-methoxyphenyl)-2-(2-pyrrolyl)-1-(4-iodobutyl)imidazole.
29. 4,5-Bis-(4-methoxyphenyl)-2-(2-pyrrolyl)-1-(3-bromopropyl)imidazole.
30. 4,5-Bis-(4-methoxyphenyl)-2-(2-pyrrolyl)-1-butylimidazole.
31. 4,5,Bis-(4-methoxyphenyl)-2-(2-pyrrolyl)-1-methylimidazole.
32. 2,3-Bis-(4-methoxyphenyl)-5,6-dihydroimidazo[1,2-a]pyrrolo-[2,1-c]pyrazine.
33. 2,3-Bis-(4-methoxyphenyl)-6,7-dihydro-5H-imidazo[1,2-a]-pyrrolo[2.1-c]-1,4-diazepine.
34. 2,3-Bis-(4-methoxyphenyl)-5,6,7,8-tetrahydroimidazo[1.2-a]-pyrrolo[2,1-c]-1,4-diazocine.
35. Pharmaceutical preparations containing one or two imidazole derivatives according to claims 1 to 34.
36. Pharmaceutical preparations according to claim 35 for the treatment of inflammatory or allergic illnesses in man.
37. Pharmaceutical preparations according to claim 35 for the treatment of migraine.
35. Pharmaceutical preparations according to claim 35 for the treatment of dysmenorrhoea.
39. Process for the preparation of imidazole derivatives of general formula 1

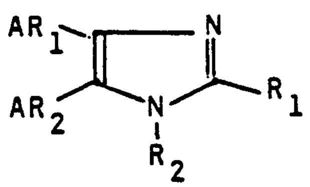

(I)

wherein
$AR_1$, $AR_2$, $R_1$ and $R_2$ have the meanings given in claim 1, characterised in that,
a diketone of general formula II

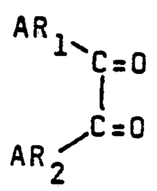

(II)

in which $AR_1$ and $AR_2$ have the meaning given above, is condensed with an aldehyde of general formula III

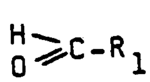

(III)

in which $R_1$ has the meaning given above, and optionally the corresponing comupond is N-alkylated.

**Revendications**

1. Dérivés de l'imidazole répondant à la formule générale I:

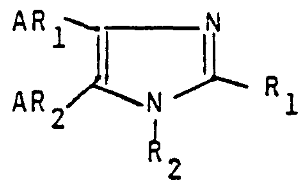

(I)

dans laquelle:

$AR_1$ et $AR_2$ représentent chacun un radical phényle éventuellement porteur d'atomes d'halogènes, de radicaux alkyles renfermant de 1 à 4 atomes de carbone ou de radicaux alcoxy renfermant de 1 à 4 atomes de carbone,

$R_1$ représente un radical pyrrolyle, un radical indolyle, un radical imidazolyle ou un radical thiazolyle, chacun de ces radicaux portant éventuellement des radicaux méthyles, méthoxycarbonyles, éthoxycarbonyles, éthoxycarbonylméthyles, éthoxycarbonyléthyles, benzyles ou benzène-sulfonyles, ou représente un radical dihydro-2,3 1H-pyrrolizinyle, et

$R_2$ représente un atome d'hydrogène, un radical alkyle contenant de 1 à 6 atomes de carbone et éventuellement porteur d'un atome d'halogène, ou un radical diméthylène, triméthylène ou tétraméthylène relié à l'atome d'azote de $R_1$.

2. Dérivés de l'imidazole de formule générale I selon la revendication 1, dérivés caractérisés en ce que les substituants $AR_1$ et $AR_2$ représentent chacun un radical phényle qui porte éventuellement, en position para, un atome de fluor ou de chlore ou un radical alcoxy contenant de 1 à 4 atomes de carbone.

3. Dérivés de l'imidazole de formule générale 1 selon la revendication 2, dérivés caractérisés en ce que les substituants $AR_1$ et $AR_2$ représentent chacun un radical phényle, fluoro-4 phényle, chloro-4 phényle ou méthoxy-4 phényle.

4. Dérivés de l'imidazole de formule générale 1 selon l'une quelconque des revendications 1 à 3, dérivés caractérisés en ce que le substituant $R_1$ est un radical pyrrolyle-2, un radical pyrrolyle-3, un radical dihydro-2,3 1H-pyrrolizinyle-7, un radical indolyle-2, un radical imidazolyle-2 ou un radical thiazolyle-2, chacun de ces radicaux portant éventuellement des radicaux méthyles, méthoxycarbonyles ou éthoxycarbonyles.

5. Bis-(méthoxy-4 phényl)-4,5 (pyrrolyl-2)-2 imidazole.

6. Bis-(chloro-4 phényl)-4,5 (pyrrolyl-2)-2 imidazole.

7. Bis-(fluoro-4 phényl)-4,5 (pyrrolyl-2)-2 imidazole.

8. Bis-(méthoxy-4 phényl)-4,5 (méthyl-1 pyrrolyl-2)-2 imidazole.

9. Bis-(méthoxy-4 phényl)-4,5 (éthoxycarbonyl-3 pyrrolyl-2)-2 imidazole.

10. Bis-(méthoxy-4 phényl)-4,5 (méthoxycarbonyl-4 pyrrolyl-2)-2 imidazole.

11. Bis-(méthoxy-4 phényl)-4,5 (benzyl-1 pyrrolyl-2)-2 imidazole.

12. Bis-(méthoxy-4 phényl)-4,5 (phénylsulfonyl-1 pyrrolyl-2)-2 imidazole.

13. Bis-(méthoxy-4 phényl)-4,5 (pyrrolyl-3)-2 imidazole.

14. Bis-(méthoxy-4 phényl)-4,5 (éthoxycarbonyl-2 pyrrolyl-3)-2 imidazole.

15. Ester éthylique de l'acide [éthoxycarbonyl-2 (bis-(méthoxy-4 phényl)-4,5 imidazolyl-2)-4 méthyl-5 pyrrolyl-3]-acétique.

16. Bis-(méthoxy-4 phényl)-4,5 (triméthyl-3,4,5 pyrrolyl-2)-2 imidazole.

17. Bis-(méthoxy-4 phényl)-4,5 (diméthyl-3,4 pyrrolyl-2)-2 imidazole.

18. Ester éthylique de l'acide [éthoxycarbonyl-2 (bis-(méthoxy-4 phényl)-4,5 imidazolyl-2)-4 méthyl-5 pyrrolyl-3]-propionate d'éthyle.

19. [Bis-(méthoxy-4 phényl)-4,5 imidazolyl-2] -7 dihydro-2,3 1H-pyrrolizine.

20. Bis-(méthoxy-4 phényl)-4,5 (indolyl-2)-2 imidazole.

21. Bis-(méthoxy-4 phényl)-4,5 (indolyl-3)-2 imidazole.

22. Bis-(méthoxy-4 phényl)-4,5 (imidazolyl-2)-2 imidazole.

23. Bis-(méthoxy-4 phényl)-4,5 (méthyl-1 imidazolyl-2)-2 imidazole.

24. Bis-(méthoxy-4 phényl)-4,5 (benzyl-1 imidazolyl-2)-2 imidazole.

25. Bis-(méthoxy-4 phényl)-4,5 (thiazolyl-2)-2 imidazole.

26. Bis-(méthoxy-4 phényl)-4,5 (pyrrolyl-2)-2 (bromo-2 éthyl)-1 imidazole.

27. Bis-(fluoro-4 phényl)-4,5 (pyrrolyl-2)-2 (bromo-3 propyl)-1 imidazole.

28. Bis-(méthoxy-4 phényl)-4,5 (pyrrolyl-2)-2 (iodo-4 butyl)-1 imidazole.

29. Bis-(méthoxy-4 phényl)-4,5 (pyrrolyl-2)-2 (bromo-3 propyl)-1 imidazole.

30. Bis-(méthoxy-4 phényl)-4,5 (pyrrolyl-2)-2 butyl-1 imidazole.

31. Bis-(méthoxy-4 phényl)-4,5 (pyrrolyl-2)-2 méthyl-1 imidazole.

32. Bis-(méthoxy-4 phényl)-2,3 dihydro-5,6 imidazo[1,2-a]pyrrolo[2,1-c-]pyrazine.

33. Bis-(méthoxy-4 phényl)-2,3 dihydro-6,7 5H-imidazo[1,2-a]pyrrolo[2,1-c]-[1,4-diazépine].

34. Bis-(méthoxy-4 phényl)-2,3 tétrahydro-5,6, 7,8 imidazo[1,2-a]pyrrolo[2,1-c]-[1,4-diazocine].

35. Compositions pharmaceutiques qui contiennent un ou deux dérivés de l'imidazole selon l'une quelconque des revendications 1 à 34.

36. Compositions pharmaceutiques selon la revendication 35 pour le traitement de maladies inflammatoires ou allergiques chez l'homme.

37. Compositions pharmaceutiques selon la revendication 35 pour le traitement de migraines.

38. Compositions pharmaceutiques selon la revendication 35 pour le traitement de dysménorrhées.

39. Procédé de préparation de dérivés de l'imidazole répondant à la formule générale I:

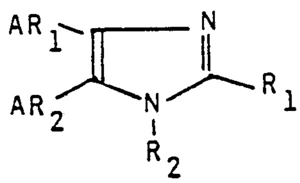

(I)

dans laquelle $AR_1$, $AR_2$, $R_1$ et $R_2$ ont les significations données à la revendication 1, procédé caractérisé en ce qu'on condense une dicétone répondant à la formule générale II:

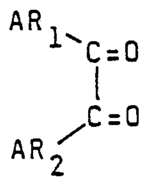

(II)

dans laquelle $AR_1$ et $AR_2$ ont les significations indiquées ci-dessus, en présence d'ions ammoniums, avec un aldéhyde répondant à la formule générale III:

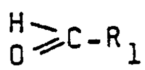

(III),

dans laquelle $R_1$ a la signification indiquée ci-dessus, et on alkyle éventuellement à l'azote le composé obtenu.